# EUROPEAN PATENT APPLICATION

(11) **EP 2 014 680 A1**
(43) Date of publication of application: **14.01.2009**
(21) Application number: 07013510.8
(22) Date of filing: 10.07.2007
(51) Int. Cl.: C07K 16/28, C07K 16/30, C07K 16/46

(54) **Recombinant, single-chain, trivalent tri-specific or bi-specific antibody derivatives**

(71) Applicant: Friedrich-Alexander-Universität Erlangen-Nürnberg, 91054 Erlangen (DE)
(72) Inventor: Singer, Heiko, 91094 Langensendelbach (DE); Kellner, Christian, 91207 Lauf (DE); Fey, Georg, H., 91077 Neunkirchen a. Br. (DE); Brünke, Jörg, 90480 Nürnberg (DE)
(74) Representative: Vossius & Partner

(57) **Abstract**

The present invention relates to a nucleic acid molecule encoding a polypeptide, wherein the polypeptide comprises (a) a first immunoglobulin domain comprising a V_{L} domain linked to a V_{H} domain, wherein the immunoglobulin domain specifically binds to an antigen expressed on tumour cells; (b) a second immunoglobulin domain comprising a V_{L} domain linked to a V_{H} domain, wherein the immunoglobulin domain specifically binds to an antigen expressed on tumour cells; and (c) a third immunoglobulin domain comprising a V_{L} domain linked to a V_{H} domain, wherein the immunoglobulin domain specifically binds to an effector cell antigen, wherein the effector cell is selected from the group consisting of NK cells, neutrophilic granulocytes, monocytes and macrophages; wherein at least one of the antigens expressed on tumour cells and bound by the immunoglobulin domain of (a) or (b) is an antigen expressed on tumour stem cells and/or on tumour precursor or progenitor cells; and wherein the ratio of immunoglobulin domains binding to antigens expressed on tumour cells and those binding to effector cell antigens is at least 2:1. The present invention furthermore relates to a vector comprising the nucleic acid molecule of the invention, a non-human host transformed with the vector of the invention, a method of producing a polypeptide comprising culturing the host of the invention under suitable conditions and isolating the polypeptide produced and a polypeptide encoded by the nucleic acid molecule of the invention or produced by the method of the invention. In addition, the present invention relates to diagnostic and pharmaceutical compositions and methods for treating tumours.

## Description

The present invention relates to a nucleic acid molecule encoding a polypeptide, wherein the polypeptide comprises (a) a first immunoglobulin domain comprising a V_{L} domain linked to a V_{H} domain, wherein the immunoglobulin domain specifically binds to an antigen expressed on tumour cells; (b) a second immunoglobulin domain comprising a V_{L} domain linked to a V_{H} domain, wherein the immunoglobulin domain specifically binds to an antigen expressed on tumour cells; and (c) a third immunoglobulin domain comprising a V_{L} domain linked to a V_{H} domain, wherein the immunoglobulin domain specifically binds to an effector cell antigen, wherein the effector cell is selected from the group consisting of NK cells, neutrophilic granulocytes, monocytes and macrophages; wherein at least one of the antigens expressed on tumour cells and bound by the immunoglobulin domain of (a) or (b) is an antigen expressed on tumour stem cells and/or on tumour precursor or progenitor cells; and wherein the ratio of immunoglobulin domains binding to antigens expressed on tumour cells and those binding to effector cell antigens is at least 2:1. The present invention furthermore relates to a vector comprising the nucleic acid molecule of the invention, a non-human host transformed with the vector of the invention, a method of producing a polypeptide comprising culturing the host of the invention under suitable conditions and isolating the polypeptide produced and a polypeptide encoded by the nucleic acid molecule of the invention or produced by the method of the invention. In addition, the present invention relates to diagnostic and pharmaceutical compositions and methods for treating tumours.

In this specification, a number of documents are cited. The disclosure content of these documents including manufacturers' manuals is herewith incorporated by reference in its entirety.

Leukaemias are cancers of the blood or bone marrow and characterized by an abnormal proliferation of blood cells, usually leukocytes. Leukaemia is clinically and pathologically split into its acute and chronic forms. *Acute leukemia* is characterized by the rapid proliferation of immature blood cells. This high number of immature cells makes the bone marrow unable to produce healthy blood cells. Acute forms of leukemia can occur in children and young adults. Immediate treatment is required in acute leukemias due to the rapid progression and accumulation of the malignant cells, which then spill over into the bloodstream and spread to other organs of the body. *Chronic leukemia* is distinguished by the excessive build up of relatively mature, but still abnormal, blood cells. Typically taking months to years to progress, the cells are produced at a much higher rate than normal cells, resulting in many abnormal white blood cells in the blood. Chronic leukemia mostly occurs in older people, but can theoretically occur in any age group. Whereas acute leukemia must be treated immediately, chronic forms are sometimes monitored for some time before treatment to ensure maximum effectiveness of therapy. The diseases are further classified according to the type of abnormal cell found most in the blood (lymphoid cells vs. myeloid cells) resulting in four main categories of leukaemia: Acute lymphocytic leukemia (also known as Acute Lymphoblastic Leukemia, or ALL), Chronic Lymphoid Leukemia (CLL), Acute Myelogenous Leukemia (also known as Acute Myeloid Leukemia, or AML) and Chronic Myelogenous Leukemia (CML).

The commonly applied therapy of cancerous diseases originating from the hematopoietic system includes irradiation and/or chemotherapy. Furthermore, under certain circumstances, the additional possibility of bone marrow transplantation is regarded suitable. However, these therapies are more or less toxic to the patient and most of the time, they do not lead to complete cure from the disease. Recently, an immunotherapy based on monoclonal antibodies has entered the clinical phase and already proven to be efficient. Prominent examples are Rituximab (anti-CD20) for the treatment of certain non-Hodgkin B-cell lymphomas (NHL) and Campath 1H (anti-CD52) in the treatment of certain chronic lymphatic leukaemias (Reff et al., 1994; Onrust et al., 1999; Riechmann et al., 1988; Hale et al., 1988). This approach has also proven to be successful in diseases such as different kinds of haemoblastosis and also solid tumours. Other antibodies are in an advanced clinical state, e.g. Epratuzumab (anti-CD22) for NHLs (Carnahan et al., 2003; Leonard et al.,2003).
An alternative to those antibodies are antibodies attached to radioactive isotopes. Examples of these antibodies are BEXXAR (anti CD20-1131) and ZEVALIN (anti CD20 Y90) (Kaminski et al., 1993; Davis et al., 2007; Cheung et al.; 2006). Instead of radioisotopes, other substances such as toxins may be used, such as done for the immunotoxin Mylotarg (anti CD33 coupled to the toxin calicheamicine) (Hamann et al., 2002; Sievers et al., 1999).
General advantages of antibodies in the treatment of cancerous diseases are their higher specificity for cancerous cells as compared to healthy cells. In particular in the case of hematologic diseases, the tumour cells are easily accessible due to their presence in the blood, bone marrow or less compact tissues.
Major drawbacks of antibodies in the above use are their size which limits their accessibility to certain tissues or their ability to penetrate tumours. Furthermore, interactions with Fc receptors of non-cytotoxic cells (e.g. B cells or platelets) or with inhibitory receptors auch as FcγRIIIb impairs their therapeutic effect (Peipp and Valerius, 2002; Clynes et al., 2000). In addition, bi-allelic polymorphisms can reduce the sensitivity to an antibody therapy, which was e.g. shown for Rituximab (Cartron et al., 2002; Weng and Levy, 2003).
Bi-specific antibodies are able to overcome some of the limitations of convential antibodies (Peipp and Valerius, 2002). Two binding sites, one for an antigen expressed on tumour cells and the other for a trigger molecule on the surface of immune effector cells (e.g. T cells, NK cells, monocytes, macrophages or granulocytes) lead to the effective recruitment of these effector cells to the tumour cells and to specific killing of the tumour cells via an antibody dependent cellular cytotoxicity (ADCC) or phagocytosis. Typically, the tumour cell lysis is induced by binding of the antibody to cytotoxic trigger molecules such as CD3 (expressed on T cells), CD16 (FcγRIIIa, expressed on NK cells), CD64 (FcγRI; expressed on activated neutrophils and monocytes/macrophages) or CD89 (FcαRI, expressed on neutrophils) (Peipp and Valerius, 2002). NK cells are known to be among the first to regenerate after stem cell transplantations, which makes them particularly suitable to eliminate cells of the minimal residual disease (MRD) (Eyrich et al., 2001; Lang et al., 2003).
Compared to previously utilized techniques, the availability of recombinant antibodies greatly facilitates the production and purification of bi-specific antibodies. Commonly known antibody formats are diabodies, minibodies, single-chain diabodies or tandem bi-specific single-chain fragments variable (bsscFv) (Peipp and Valerius, 2002), the two latter of which consist of only one molecule.

The antigen(s) chosen to be bound by the therapeutic antibody derivative is/are crucial for the therapy. Antibodies binding to CD19 expressed on B-cells have been described before in the treatment of B lymphoid neoplasias (Grossbard et al., 1992). CD19 is expressed on B cells in all stages of their development except for the mature plasma cell. Until now, this antigen has not been found on haematopoietic stem cells or other cells beyond the B lymphoid compartment. The formats used so far comprised whole antibodies, immunoconjugates, immunoliposomes, immunotoxins and bi-specific formats. The treatment of six patients with a murine CD19 IgG2A antibody led to a partial remission in one patient (Hekmann et al., 1991). A glycol-engineered chimeric CD19 antibody was capable of mediating ADCC via NK cells (Barbin et al., 2006), a further chimeric CD19 antibody decelerated the growth of a CD19-positive tumour in a SCID-mouse model Pietersz et al., 1995). Anti-CD19 antibody derivatives coupled to the tyrosine kinase inhibitor genistein as well as to radionuclides, cytotoxins or exotoxins were developed which all showed an amelioration of the cancerous condition (Messinger et al., 1998 ; Vervoordeldonk et al., 1994; Vallera et al., 2004; Schwemmlein et al., 2007). Bi-specific antibodies known are e.g. those directed against CD19 and CD64 using interferon-stimulated macrophages as effector cells (Ely et al., 1996). Antibody derivatives directed against CD19 and CD3 (Haagen et al., 1992; Weiner and de Gast, 1995; Kiprianov et al, 1998; Loffler et al., 2000) as well as CD19 and CD16 (Kiprianov et al., 2002) activate cytotoxic T-cells or NK cells, respectively. Although cell culture data demonstrated significant lytic activity for the [CD19xCD3] bi-specific antibody derivatives, the cross-linking of CD3 mediated by the antibody led to nonspecific T-cell activation, causing a toxicity to the cell associated with the antibody (Segal et al., 1999). [CD19xCD16] antibody derivatives have been produced as single-chain Fv molecule, as double-chain diabody and as tetravalent diabody and have been proven to be effective in a mouse model of a human lymphoma (EP 1314741; Affimed Therapeutics AG): CD16 is the low affinity receptor for IgG (FcγRIII), which is constitutively expressed as a transmembrane isoform on the surface of NK cells and macrophages (CD16a), and as a glycosyl phosphatidyl inositol (GPI)-anchored molecule on the surface of neutrophils (CD16b) (Ravetch and Kinet, 1991; van deWinkel and Anderson, 1991). For intracellular signalling, CD16a requires association with the FcRγ chain containing the immunoreceptor tyrosine-based activation motif (ITAM), which triggers downstream signalling. Studies with conventionally coupled bi-specific antibodies redirecting NK cells via CD16 demonstrated potent cytolysis of cultured malignant cells and in animal models (Garcia de Palazzo et al, 1992; Hombach et al, 1993; Kipriyanov et al, 2002). Therefore, clinical trials with CD16-directed bi-specific antibodies were initiated (Weiner et al, 1995; Hartmann et al, 1997). However, immunogenicity of hybrid-hybridoma antibodies, as well as undesired side effects caused by the presence of Fc-domains, and difficulties in producing sufficient amounts of clinical- grade material limited these clinical trials. Another bi-specific anti[CD19xCD16] antibody utilizes two single chain Fvs combined in one polypeptide chain (Bruenke et al., 2005).

The effectiveness of antibodies depends on their stability in human serum, their location in the organism and their affinity. Several means have been developed to influence these properties. For example, a disulfide bridge between the two variable domains of an scFv antibody can greatly improve the serum stability but very often results in insoluble expression of the recombinant protein or leads to a reduction of affinity. Further measures applicable for improvement of pharmacokinetical properties are to couple the antibody derivative to polyethyleneglycol (PEG), components binding to human serum albumin (HSA) or HSA itself to the antibody derivative.
Finally, the affinity to the targeted tumour cells could be enhanced by developing multivalent antibody derivatives. Published formats comprise minibodies, tribodies, tetravalent diabodies or trivalent triabodies, which are equipped with one or more specificities Their reduced size allows for tumour penetration as well as for a longer retention time in the organism since their molecular weight lies still above the exclusion barrier of the kidneys. One remaining disadvantage of the molecules comprising more than two antigen binding sites is that they still consist of at least two polypeptide chains since until now it has not been described that a single-chain molecule with more than two specificities could be obtained which is applicable as a therapeutic. If an antibody derivative consists of more than one polypeptide chain it is more difficult to produce and purify this derivative to obtain a homogenous mixture.
Finally, the similar affinity of antibody derivatives with the same number of specific binding sites for both tumour cells and effector cells often leads to the activation of the latter without being recruited to a tumour or other cell before, which leads to a reduced cytotoxic potential of the effector cell.

The present invention provides novel and advantageous antibody derivatives suitable as therapeutic in haematologic diseases having a higher affinity for tumour cells than for effector cells thus shifting the avidity towards tumour cells and being more stable.

Accordingly, the present invention relates to a nucleic acid molecule encoding a polypeptide, wherein the polypeptide comprises (a) a first immunoglobulin domain comprising a V_{L} domain linked to a V_{H} domain, wherein the immunoglobulin domain specifically binds to an antigen expressed on tumour cells; (b) second immunoglobulin domain comprising a V_{L} domain linked to a V_{H} domain, wherein the immunoglobulin domain specifically binds to an antigen expressed on tumour cells; and (c) a third immunoglobulin domain comprising a V_{L} domain linked to a V_{H} domain, wherein the immunoglobulin domain specifically binds to an effector cell antigen, wherein the effector cell is selected from the group consisting of NK cells, neutrophilic granulocytes, monocytes and macrophages; wherein at least one of the antigens expressed on tumour cells and bound by the immunoglobulin domain of (a) or (b) is an antigen expressed on tumour stem cells or on tumour precursor or progenitor cells; and wherein the ratio of immunoglobulin domains binding to antigens expressed on tumour cells and those binding to effector cell antigens is at least 2:1.

In accordance with the present invention the term "nucleic acid molecule" defines a linear molecular chain consisting of more than 30 nucleotides. The group of molecules here designated as "nucleic acid molecules" also comprises complete genes.

"Nucleic acid molecules", in accordance with the present invention, include DNA, such as cDNA or genomic DNA, and RNA. Further included are nucleic acid mimicking molecules known in the art such as synthetic or semi-synthetic derivatives of DNA or RNA and mixed polymers. Such nucleic acid mimicking molecules or nucleic acid derivatives according to the invention include phosphorothioate nucleic acid, phosphoramidate nucleic acid, 2'-O-methoxyethyl ribonucleic acid, morpholino nucleic acid, hexitol nucleic acid (HNA) and locked nucleic acid (LNA) (see Braasch and Corey, Chem Biol 2001, 8: 1). LNA is an RNA derivative in which the ribose ring is constrained by a methylene linkage between the 2'-oxygen and the 4'-carbon. They may contain additional non-natural or derivative nucleotide bases, as will be readily appreciated by those skilled in the art.

The present invention furthermore contemplates nucleic acid molecules complementary to the above-defined nucleic acid molecule as well as nucleic acid molecules hybridizing thereto under stringent conditions.
"Stringent conditions" refers to hybridization conditions which comprise, e.g. an overnight incubation at 65°C in 4x SSC (600 mM NaCl, 60 mM sodium citrate) followed by washing at 65°C in 0.1x SSC for one hour. Alternatively, hybridization conditions can comprise: an overnight incubation at 42°C in a solution comprising 50% formamide, 5x SSC (750 mM NaCl, 75 mM sodium citrate), 50 mM sodium phosphate (pH 7.6), 5x Denhardt's solution, 10% dextran sulphate, and 20 µg/ml denatured, sheared salmon sperm DNA, followed by washing in e.g. 0.1-0.5x SSC at about 55-65°C for about 5 to 20 min. Said conditions for hybridization are also known by a person skilled in the art as "highly stringent conditions for hybridization".

The term "polypeptide" as used herein describes linear molecular chains of amino acids, including single chain proteins or their fragments, containing 30 or more amino acids. Furthermore, peptidomimetics of such proteins/polypeptides where amino acid(s) and/or peptide bond(s) have been replaced by functional analogues are also encompassed by the invention. Such functional analogues include all known amino acids other than the 20 gene-encoded amino acids, such as selenocysteine. The terms "polypeptide" and "protein", which are used interchangeably, also refer to naturally modified polypeptides/proteins where the modification is effected e.g. by glycosylation, acetylation, phosphorylation and similar modifications which are well known in the art.

The term "immunoglobulin domain comprising a V_{L} domain linked to a V_{H} domain" refers to single-chain fragment variable domains of immunoglobulins (scFv) which have been shown to be necessary and sufficient to bind their antigen. Each of the V_{H} or V_{L} domains comprises three complementarity determining regions (CDRs), highly variable regions mainly responsible for the binding of the antigen. Where applicable, a V_{L} or V_{H} domain may consist of at least one CDR per domain, preferably at least two, preferably all three, as long as the resulting immunoglobulin domain exerts the desired function, i.e. specifically binds to its target antigen. The nucleic acid molecule of the invention may encode immunoglobulin domains derived from a single species, but also chimeric or humanized molecules.

The term "specifically binds " or "specifically binding" (having the same meaning as "specifically interacting") as used in accordance with the present invention means that the immunoglobulin domain does not or essentially does not cross-react with an epitope with a structure similar to that of the target antigen. Cross-reactivity of a panel of immunoglobulin domains under investigation may be tested, for example, by assessing binding of said panel of immunoglobulin domains under conventional conditions to the epitope of interest as well as to a number of more or less (structurally and/or functionally) closely related epitopes. Only those immunoglobulin domains that bind to the epitope of interest in its relevant context (e.g. a specific motif in the structure of a protein) but do not or do not essentially bind to any of the other epitopes are considered specific for the epitope of interest and thus to be immunoglobulin domains in accordance with this invention. Corresponding methods are described e.g. in Harlow and Lane, 1988 and 1999, loc cit.

The term "antigen expressed on tumour cells" refers to antigens expressed on the surface of tumour cells. "Tumour cells" in accordance with the present invention are transformed cells that are characteristic of or give rise to tumours. The term "tumour" in accordance with the present invention refers not only to solid tumours, but also to non-solid tumourous conditions such as, for example, leukaemia.

An "effector cell antigen" is an antigen expressed on the surface of effector cells. The term "effector cells" as used throughout the present invention refers to terminally differentiated leukocytes that perform one or more specific functions in the immune system. "Effector cells" in accordance with the present invention are NK cells, granulocytes, macrophages or monocytes. It is preferred that the effector cells are NK cells.

The term "stem cells" in accordance with the present invention refers to primal cells found in all multi-cellular organisms which retain the ability to renew themselves through mitotic cell division and can differentiate into a diverse range of specialized cell types. Hematopoietic stem cells (HSCs) are a small subset of bone marrow cells, representing approximately 1 : 10⁵ - 1 : 10⁶ cells, with the unique ability of generating all other mature cell types of the hematopoietic system. They have the defining characteristic ability to divide in both a symmetric and asymmetric manner, and to generate lineage committed progeny cells. HSCs rarely divide but have a high self-renewal capability. The lineage-committed daughter cells are the "progenitor" and "precursor cells", which proliferate more actively (Spangrude et al., 1988; Bryder et al., 2006). The term "precursor or progenitor cell" refers to immature or undifferentiated cells, typically found in post-natal animals. The term "Tumour pecursor or progenitor cells" in accordance with the present invention refers to cells with altered properties giving rise to tumour cells. Examples for such tumour precursor or progenitor cells are e.g. leukaemic precursor or progenitor cells. Progenitor cells share many common features with stem cells. Like stem cells, progenitor cells have a capacity for self-renewal and differentiation, although these properties may be limited compared with those of stem cells. Progenitor or precursor cells can be seen as intermediate progeny of stem cells still capable of proliferating.

The ratio of immunoglobulin domains binding to antigens expressed on tumour cells and those binding to effector cell antigens is an essential feature of the present invention. As discussed above, previously known antibody derivatives comprise bi- or tri- specific derivatives of multi-chain immunoglobulins or bi-specific derivatives of single-chain Fvs. To the best knowledge of the applicant, optimization of the ratio of binding sites for tumour antigens and effector cell antigens and its effect on the quality of the immune response against the tumour cells has so far not been published. The present invention for the first time discloses a ratio of at least 2:1 of immunoglobulin domains binding to tumour cell antigens and effector cells antigens. The higher fraction of immunoglobulin domains binding to antigens expressed on tumour cells has the effect that the affinity for the tumour cells is increased. Thus, the higher number of antigen binding moieties targeted to antigen expressed on tumour cells increases the probability that the polypeptide of the invention binds to a tumour cell before it binds to an effector cell. The recruitment of tumour cells prior to the effector cells is advantageous for the following reason: Up to now, in a therapeutic context, immune responses are induced upon binding of an immunoglobulin with one or two specificities or a natural trigger molecule to a respective antigen expressed on the surface of effector cells mediating an immune response. Thereby, the effector cells cannot distinguish whether an antibody molecule is bound to a tumour cell or not leading to an unspecific immune response in case the antibody derivative is not bound to a tumour cell. In contrast, the 2:1 ratio of antigen binding sites specific for tumour antigens and those specific for effector cells enhance the probability that the antibody derivative of the present invention binds to a tumour cell before it binds to an antigen of an effector cell. This greatly reduces the amount of unspecific immune responses and may thus decrease adverse side-effects of previously known molecules.
It is expected that ratios greater than 2:1, e.g. 3:1 or 4:1 which are also contemplated in the present invention exert the same or even more advantageous properties.

Apart from the effects described above, the present invention for the first time demonstrates that a single chain polypeptide comprising three antigen binding specificities in the format of scFvs can be recombinantly and solubly expressed and exerts the desired effects. As described above, bi-specific single-chain Fv antibodies were known in the prior art. However, it was not generally believed that a tri-specific single-chain molecule which is supposed to be structurally complex, could be obtained which is correctly folded, stable and still has the desired function, i.e. it binds its target antigens, whereby the avidity for tumour cell antigens is higher than that for effector cells. A further advantage of the format of the polypeptide of the present invention is that the combination of three scFvs in one polypeptide reduces the surface of the molecule thus reducing potential immunogenicity in the case of antibodies derived from other organisms than the one to be cured from a tumour.

In a preferred embodiment, the immunoglobulin domain of items (a) and (b) bind to the same antigen expressed on tumour cells. For example, both immunoglobulin domains may bind to CD19, CD33, CD13, CD44var or CD123.

In another preferred embodiment, the immunoglobulin domain of items (a) and (b) bind to different antigens expressed on tumour cells.

In a further preferred embodiment, the at least one antigen expressed on tumour stem cells or tumour precursor or progenitor cells is CD19, CD33, CD13, CD44var or CD123.

In an additional preferred embodiment of the present invention, the antigens expressed on tumour cells, preferably on tumour stem cells or tumour precursor or progenitor cells, and bound by the immunoglobulin domains of (a) and (b) are CD19 and CD33, CD19 and CD13, CD19 and CD123, CD19 and CD44var, CD33 and CD13, CD33 and CD123, CD33 and CD44var, CD13 and CD123, CD13 and CD44var or CD123 and CD44var.

In general, on healthy cells only one type of the above antigens is expressed at a time. However, in certain tumours, particularly in certain leukaemias, more than one of the above or other specific antigens may be expressed at the same time. Prominent examples described in the prior art are CD13 and CD19, CD33 and CD19, as well as CD19 and MCSP (Melanoma-associated Chondroitin Sulfate Proteoglycan), being co-expressed in be-phenotypic or mixed lineage leukaemias (Greil *et al.,* 1994; Hrusak and Porwit-MacDonald, 2002; Casasnovas *et al*., 2003; Haagen *et al.,* 1992). The polypeptide encoded by the nucleic acid molecule of the present invention and having a specificity for two different antigens usually not expressed simultaneously on healthy cells has an increased affinity for aberrant cells, i.e. tumour cells simultaneously expressing two of the above antigens This enhances the selectivity of the molecule for the tumour cells.

In another preferred embodiment, the effector cell antigen bound by the immunoglobulin domain of (c) is CD16 (FcγRIIIa) or CD64 (FcγRI). Further antigens suitable in the context of the present invention are CD89, NKG2D, NKp30, NKp44 or NKp46.

In a further preferred embodiment, at least one immunoglobulin domain comprises at least one cysteine residue capable of forming intramolecular disulfide bridges.

It is known that intramolecular cysteine bridges stabilize an antibody-fragment and positively influence its serum stability and/or in some cases its binding affinity for the target epitope etc. Disulfide bridges are generally found in naturally occurring antibodies, where they are present in the variable and constant regions. Many scFv antibodies were found to have a lower affinity for antigen than the Fab counterpart which was attributed to the fact that the peptide linker of the scFv interferes with binding (Batra et al., 1992; Pantoliano et al., 1991; Stemmer et al., 1993). In order to improve the performance of scFv antibodies with different numbers of binding sites, it was attempted to introduce cysteins capable of forming disulfide bridges at positions in the molecule where they do not naturally occur. The following prerequisites have to be met when designing a modified antibody with artificial cysteine bridges. First, the disulfide bridge should connect amino acids in structurally conserved regions of the Fv, i.e. in the V_{H} and V_{L} regions. Furthermore, the distance between the V_{H} and the V_{L} domain should be small enough to enable the formation of a disulfide bridge without the generation of adverse strain in the Fv molecule. Finally, the disulfide bridge should be distant enough from the CDRs to not interfere with antigen binding. As a consequence, cysteines introduced to form the disulfide bridge are in the framework regions of the Fv which connect,the CDRs of the V_{H} and V_{L} regions at positions that are structurally conserved in most antibodies. Glockshuber et al. (1990), Brinkmann et al. (1993) and Reiter et al. (1994) set out these criteria and successfully established the first disulfide stabilized Fv antibodies.
However, most of the trials undertaken to stabilize antibodies comprising only variable regions in this way as in scFvs encountered various difficulties, i.e. similar problems were faced during expression of the modified antibodies as compared to the expression of recombinant whole antibodies. For example, the resulting molecules turned out to be insoluble when recombinantly expressed, which is probably due to uncontrolled disulfide bridge formation and aggregation resulting therefrom. Apart from that, disulfide stabilized scFvs also often displayed a reduced affinity to the antigen as compared to their non disulfide stabilized counterparts.
The present invention could overcome this problem. Although predicted to result in similar difficulties as described above by various experts in the field, the immunoglobulin domains of the antibody derivative of the present invention could be stabilized by intradomain cysteine bridges without at the same time turning the whole molecule insoluble. Mutations in the amino acid sequence of the polynucleotide of the invention can e.g. be introduced according to Reiter et al. (1994) at base triplets at position 100 in the V_{L} domain or at position 44 in the V_{H} domain, so that they encode a cysteine residue. All positions are according to the Kabat numbering (Kabat et al., 1991).
In the context of the present invention, immunoglobulin domains binding to certain antigens are stabilized by mutating two appropriate amino acids as described above to cysteins. The introduction of disulfide bridges into immunoglobulin domains of scFvsspecific for CD19 and CD16 was shown to greatly enhance the stability of the molecules, without affecting their specificity or reducing their affinity, which was observed for other scFvs.

Means to introduce mutations into a nucleic acid molecule are known to the skilled person and can e.g. be retrieved from in Sambrook, 2001 and Ausubel, 2001.

In a different preferred embodiment, the nucleic acid molecule of the invention encodes at least one linker which separates at least one V_{H} from a V_{L} domain or at least one V_{L} from a V_{H} domain.

The term "linker" as used in accordance with the present invention relates to a sequel of amino acids linking either the V_{H} and V_{L} domains of one immunoglobulin domain or two immunoglobulin domains. Thus; the linker may separate at least one V_{H} and V_{L} region forming one immunoglobulin domains and/or the linker may separate at least one V_{L} and V_{H} region of neighbouring immunoglobulin domains.

Linkers as envisaged by the present invention are (poly)peptide linkers of 1 to 40 amino acids in length. Preferably, the linkers are of 5 to 25 amino acids in length. Even more preferably, the linkers are 10 to 20 amino acids in length. The linkers separating the V_{H} and V_{L} domains of one immunoglobulin domain and the ones separating the different immunoglobulin domains can have the same or different lengths and may comprise the same or a different amino acid sequence. Preferably, the linkers have the same length and the same amino acid sequence.

In another preferred embodiment, the linkers separating the immunoglobulin domains differ in length and/or amino acid sequence from those separating the V_{H} and V_{L} regions within an immunoglobulin domain. For example, the former could be longer and designed in order to promote the flexibility of the immunoglobulin domains towards each other or to facilitate correct folding of the molecule and/or enhance the affinity of one immunoglobulin domain to its target antigen. Furthermore, the nature, i.e. the length and/or amino acid sequence of the linker may modify or enhance the stability and/or the solubility of the molecule.
The length and sequence of a linker depends on the composition of the V_{H} and V_{L} domains forming an immunoglobulin domain. For example, starting from the N-terminus of the polypeptide of the invention or from the 5'-end of the nucleic acid molecule of the invention, if the V_{L} domain is followed by the V_{H} domain, linkers of 20 amino acid separating the V regions of one immunoglobulin domain may be optimal. In contrast, if the V_{H} domain is followed by the V_{L} domain, the respective linker my have an optimal length of 15 amino acids. Without wishing to be bound by any scientific theory, it is believed that these differences may be due to sterical reasons leading to linkers of different lengths promoting the correct folding of immunoglobulin domains having a different arrangement of V domains.
The skilled person is well aware of methods to test the suitability of different linkers within or between immunoglobulin domains. For example, the properties of the molecule can easily be tested by comparing the binding affinity of the immunoglobulin domain. In case of the tri-specific molecules of the invention, the respective measurements for each immunoglobulin domain may be carried out. The stability of the resulting molecule can be measured - using a flow cytometry based method to determine the residual binding capacity of the molecule after incubation in human serum at 37°C for several time periods. Other suitable tests can e.g. be found in Bruenke et al.(2005).

In another preferred embodiment no linker is present between at least one V_{H} and V_{L} domain or V_{L} and V_{H} domain within or between immunoglobulin domains.

In another preferred embodiment, the nucleic acid molecule of the invention encodes two immunoglobulin domains specifically binding to CD19 and one immunoglobulin domain specifically binding to CD16 (FcγRIIIa).

An antibody encoded by the nucleic acid molecule of the invention comprising two binding sites for a tumour antigen and one for the effecter antigen exerts superior properties as compared to the bi-specific scFv antibody derivative. The novel antibody derivative of the present invention exhibits in Antibody dependent cellular cytotoxicity (ADCC) assays a higher efficacy as compared to the bi-specific scFv derivative. Furthermore, the single chain Fv Triabody has more favourable pharmacokinetical properties. In mice the in vivo serum retention half-life is higher as the half life displayed by the tandem bi-specific scFv antibody. Furthermore, a disulfide stabilized antibody having disulfide bridges in all immunoglobulin domains was shown previously to be more stable as compared to a non disulfide-stabilized variant of the bi-specific scFv antibody (Bruenke *et al.,* 2005).

In yet another preferred embodiment, the polypeptide further comprises at least one (poly)peptide unrelated to immunoglobulin domains which can be e.g. a tag or a functional (poly)peptide suitable to improve the performance of the polypeptide of the invention. The tag can e.g. be a Strep-tag, a His-tag, a Myc-tag or a Flag-tag. Functional polypeptide are e.g. a kappa secretion leader, human serum albumin (hsa) or fragments thereof, peptides capable of binding to hsa or other serum proteins; peptides capable of binding to neonatal Fc receptor (FcRn), human muscle aldolase (hma) or fragments thereof, CD8 hinge region, immunoglobulin constant regions, Interleukin-2, Interleukin-15 and Interleukin-18, Granulocyte-Macrophage-Colony Stimulating Factor (GM-CSF) and Granulocyte Stimulating Factor (G-CSF).

The term "(poly)peptide" as used herein describes a group of molecules which comprises the group of peptides, consisting of up to 30 amino acids, as well as the group of polypeptides, consisting of more than 30 amino acids, as defined above.

The term "fragments thereof" in connection with the present invention refers to fragments of proteins still having one or more of the biological functions of the full-length (poly)peptide. In particular, the fragments of (poly)peptides as envisaged in the present invention are capable of increasing the stability and/or the serum half-life of the antibody derivative of the present invention.
It is well known in the art that functional polypeptides may be cleaved to yield fragments with unaltered or substantially unaltered function. Such cleavage may include the removal of a given number of N- and/or C-terminal amino acids. Additionally or alternatively, a number of internal (non-terminal) amino acids may be removed, provided the obtained polypeptide has the function of the full length (poly)peptide. Said number of amino acids to be removed from the termini and/or internal regions may be one, two, three, four, five, six, seven, eight, nine, ten, 15, 20, 25, 30, 40, 50 or more than 50. Any other number between one and 50 is also deliberately envisaged in accordance with this invention.
Means and methods for determining such functional domains of polypeptides are well known in the art and include experimental and bioinformatic means. Experimental means include the systematic generation of deletion mutants and their assessment in assays for the desired functions above known in the art.

Bioinformatic means include database searches. Suitable databases included protein sequence databases. In this case a multiple sequence alignment of significant hits is indicative of domain boundaries, wherein the domain(s) is/are comprised of the/those subsequences exhibiting an elevated level of sequence conservation as compared to the remainder of the sequence. Further suitable databases include databases of statistical models of conserved protein domains such as Pfam maintained by the Sanger Institute, UK (www.sanger.ac.uk/Software/Pfam).

Some of the (poly)peptides to be further encoded by the nucleic acid molecule of the present invention may facilitate the purification of the recombinantly expressed polypeptide, e.g. various tags. Methods to add tags and/or other polypeptides to the polypeptide encoded by the nucleic acid molecule of the present invention are well known to the skilled person and described e.g. in Sambrook, 2001.

In a preferred embodiment, the tumour cells of the invention are preferably leukaemia or lymphoma cells. Depending on the origin and the stage of the lymphoid or myeloid cells the leukaemia originates from, different patterns of antigens are expressed on their surface. Antigens expressed on the surface of cells derived from the B-cell lineage are e.g. CD19, CD20 or CD22, whereas antigens typical for cells derived from myeloid cells are e.g. CD13, CD33 or CD123. These antigens are not exclusively expressed on leukaemia cells but also on healthy cells (Taussig et al., 2005). CD44v6 is expressed on a subset of both lymphoid and myeloid precursor cells and is strongly over-expressed on AML cells and AML leukemia stem cells (LSCs) (Jin et al., 2006; Legras et al., 1998).
In another preferred embodiment, the tumour stem cells are leukaemic stem cells. "Leukemic stem cells" (LSC) in accordance with the present invention are a population of malignantly transformed cells, which drive the development of leukemias in a similar manner as HSCs drive the generation of normal hematopoietic cells. They represent a small minority of all leukaemic cells (approx. 1 : 50,000 - 1 : 10⁶). Their defining properties are the ability of indefinite self-renewal and the ability to divide both in a symmetric and asymmetric manner. The sum of all leukemic cells in one patient is therefore not a homogeneous population of cells, but a heterogeneous mixture consisting of leukemic stem cells, intermediate progenitors and the bulk of more differentiated leukemic blast cells. The LSCs have a different composition of cell surface markers than the buk leukemia cells and are highly resistant to chemotherapeutics. They often survive chemotherapy, which eliminates the bulk of tumour cells and generates a remission. The remaining few LSCs are among the minimal residual disease (MRD) cells, which fuel the repopulation of the leukemia mass ("relapse") (Tan et al., 2006; Bonnet and Dick, 1997; Lapidot et al., 1994; Passegue et al., 2003).

In another preferred embodiment the tumour precursor or progenitor cells are leukaemic precursor or progenitor cells.

In a further preferred embodiment, the tumour is a leukaemia or lymphoma. As described above, Leukaemias are cancers of the blood or bone marrow and are characterized by an abnormal proliferation of blood cells, usually leukocytes. Leukaemias can develop at different stages during developmet, including the stem cell stage (Castor et al., 2005).

In a different aspect, the present invention relates to a vector comprising the nucleic acid molecule of the present invention.

Preferably, the vector is a plasmid, cosmid, virus, bacteriophage or another vector used e.g. conventionally in genetic engineering.

The nucleic acid molecule of the present invention may be inserted into several commercially available vectors. Non-limiting examples include prokaryotic plasmid vectors, such as the pUC-series, pBluescript (Stratagene), the pET-series of expression vectors (Novagen) or pCRTOPO (Invitrogen) and vectors compatible with expression in mammalian cells like pREP (Invitrogen), pcDNA3 (Invitrogen), pCEP4 (Invitrogen), pMC1neo (Stratagene), pXT1 (Stratagene), pSG5 (Stratagene), EBO-pSV2neo, pBPV-1, pdBPVMMTneo, pRSVgpt, pRSVneo, pSV2-dhfr, pIZD35, pLXIN, pSIR (Clontech), pIRES-EGFP (Clontech), pEAK-10 (Edge Biosystems) pTriEx-Hygro (Novagen) and pCINeo (Promega). Examples for plasmid vectors suitable for Pichia pastoris comprise e.g. the plasmids pAO815, pPIC9K and pPIC3.5K (all Intvitrogen).

The nucleic acid molecule of the present invention referred to above may also be inserted into vectors such that a translational fusion with another nucleic acid molecule is generated. The other nucleic acid molecules may encode a protein which may e.g. increase the solubility and/or facilitate the purifcation of the protein encoded by the nucleic acid molecule of the invention. Non-limiting examples include pET32, pET41, pET43. The vectors may also contain an additional expressible polynucleotide coding for one or more chaperones to facilitate correct protein folding. Suitable bacterial expression hosts comprise e. g. strains derived from BL21 (such as BL21(DE3), BL21(DE3)PlysS, BL21(DE3)RIL, BL21(DE3)PRARE) or Rosetta®.

For vector modification techniques, see Sambrook and Russel, 2001. Generally, vectors can contain one or more origin of replication (ori) and inheritance systems for cloning or expression, one or more markers for selection in the host, e. g., antibiotic resistance, and one or more expression cassettes. Suitable origins of replication (ori) include, for example, the Col E1, the SV40 viral and the M 13 origins of replication.

The coding sequences inserted in the vector can e.g. be synthesized by standard methods, or isolated from natural sources. Ligation of the coding sequences to transcriptional regulatory elements and/or to other amino acid encoding sequences can be carried out using established methods. Transcriptional regulatory elements (parts of an expression cassette) ensuring expression in prokaryotes or eukaryotic cells are well known to those skilled in the art. These elements comprise regulatory sequences ensuring the initiation of the transcription (e. g., translation initiation codon, promoters, enhancers, and/or insulators), internal ribosomal entry sites (IRES) (Owens *et al.,* 2001) and optionally poly-A signals ensuring termination of transcription and stabilization of the transcript. Additional regulatory elements may include transcriptional as well as translational enhancers, and/or naturally-associated or heterologous promoter regions. Preferably, the nucleic acid molecule of the invention is operably linked to such expression control sequences allowing expression in prokaryotes or eukaryotic cells. The vector may further comprise nucleotide sequences encoding secretion signals as further regulatory elements. Such sequences are well known to the person skilled in the art: Furthermore, depending on the expression system used, leader sequences capable of directing the expressed polypeptide to a cellular compartment may be added to the coding sequence of the polynucleotide of the invention. Such leader sequences are well known in the art.

Specifically-designed vectors allow the shuttling of DNA between different hosts, such as bacteria- fungal cells or bacteria-animal cells.
An expression vector according to this invention is capable of directing the replication, and the expression, of the polynucleotide and encoded enzyme of this invention. Suitable expression vectors which comprise the described regulatory elements are known in the art such as Okayama-Berg cDNA expression vector pcDV1 (Pharmacia), pRc/CMV, pcDNA1, pcDNA3 (In-Vitrogene, as used, inter alia in the appended examples), pSPORT1 (GIBCO BRL) or pGEMHE (Promega), or prokaryotic expression vectors, such as lambda gt11, pJOE, the pBBR1-MCS - series, pJB861, pBSMuL, pBC2, pUCPKS, pTACT1 or, preferably, the pET vector (Novagen).

The nucleic acid molecules of the invention as described herein above may be designed for direct introduction or for introduction via liposomes, phage vectors or viral vectors (e.g. adenoviral, retroviral) into the cell. Additionally, baculoviral systems or systems based on Vaccinia Virus or Semliki Forest Virus can be used as eukaryotic expression system for the nucleic acid molecules of the invention. Expression vectors derived from viruses such as retroviruses, vaccinia virus, adeno-associated virus, herpes viruses, or bovine papilloma virus, may be used for delivery of the polynucleotides or vector into targeted cell population. Methods which are well known to those skilled in the art can be used to construct recombinant viral vectors; see, for example, the techniques described in Sambrook, 2001 and Ausubel, 2001.

A typical mammalian expression vector contains the promoter element, which mediates the initiation of transcription of mRNA, the protein coding sequence, and signals required for the termination of transcription and polyadenylation of the transcript. Moreover, elements such as origin of replication, drug resistance gene, regulators (as part of an inducible promoter) may also be included. The *lac* promoter is a typical inducible promoter, useful for prokaryotic cells, which can be induced using the lactose analogue *isopropylthiol-b-D-galactoside.* ("IPTG"). For recombinant expression, the antibody fragment may be ligated between e.g. the PeIB leader signal, which directs the recombinant protein in the periplasm and the gene III in a phagemid called pHEN4 (described in Ghahroudi *et al,* 1997). Additional elements might include enhancers, Kozak sequences and intervening sequences flanked by donor and acceptor sites for RNA splicing. Highly efficient transcription can be achieved with the early and late promoters from SV40, the long terminal repeats (LTRs) from retroviruses, e.g., RSV, HTLVI, HIVI, and the early promoter of the cytomegalovirus (CMV). However, cellular elements can also be used (e.g., the human actin promoter). Suitable expression vectors for use in practicing the present invention include, for example, vectors such as pSVL and pMSG (Pharmacia, Uppsala, Sweden), pRSVcat (ATCC 37152), pSV2dhfr (ATCC 37146) and pBC12MI (ATCC 67109). Mammalian host cells that could be used include, human Hela, 293, H9 and Jurkat cells, mouse NIH3T3 and C127 cells, Cos 1, Cos 7 and CV1, quail QC1-3 cells, mouse L cells and Chinese hamster ovary (CHO) cells. Alternatively, the recombinant (poly)peptide can be expressed in stable cell lines that contain the gene construct integrated into a chromosome. The co-transfection with a selectable marker such as dhfr, gpt, neomycin, hygromycin allows the identification and isolation of the transfected cells. The transfected nucleic acid can also be amplified to express large amounts of the encoded (poly)peptide. The DHFR (dihydrofolate reductase) marker is useful to develop cell lines that carry several hundred or even several thousand copies of the gene of interest. Another useful selection marker is the enzyme glutamine synthase (GS) (Murphy et al. 1991; Bebbington et al. 1992). Using these markers, the mammalian cells are grown in selective medium and the cells with the highest resistance are selected. As indicated above, the expression vectors will preferably include at least one selectable marker. Such markers include dihydrofolate reductase, G418 or neomycin resistance for eukaryotic cell culture and tetracycline, kanamycin or ampicillin resistance genes for culturing in *E*. *coli* and other bacteria. Representative examples of appropriate hosts include, but are not limited to, bacterial cells, such as *E*. *coli,* Streptomyces and *Salmonella typhimurium* cells; fungal cells, such as yeast cells; insect cells such as Drosophila S2 and Spodoptera Sf9 cells; animal cells such as CHO, COS, 293 and Bowes melanoma cells; and plant cells. Appropriate culture mediums and conditions for the above-described host cells are known in the art.

Possible regulatory elements permitting expression in prokaryotic host cells comprise, e.g., the *lac*, *trp* or *tac* promoter, the lacUV5 or the trp promotor in *E*. *coli*, and examples for regulatory elements permitting expression in eukaryotic host cells (the more preferred embodiment) are the *AOX1* or *GAL1* promoter in yeast or the CMV- (Cytomegalovirus), SV40-, RSV-promoter (Rous sarcoma virus), the gai10 promoter, human elongation factor 1α-promoter, CMV enhancer, CaM-kinase promoter, the Autographa californica multiple nuclear polyhedrosis virus (AcMNPV) polyhedral promoter or a globin intron in mammalian and other animal cells. Preferred promoters are natural immunoglobulin promoters. Besides elements which are responsible for the initiation of transcription such regulatory elements may also comprise transcription termination signals, such as the SV40-poly-A site or the tk-poly-A site or the SV40, lacZ and AcMNPV polyhedral polyadenylation signals, downstream of the polynucleotide.

In yet another aspect, the present invention relates to a non-human host transformed with the vector of the invention.

Suitable prokaryotic hosts comprise e.g. bacteria of the species Escherichia or Bacillus. Suitable eukaryotic host cells are e.g. yeasts such as Saccharomyces cerevisiae or Pichia pastoris.
Mammalian host cells that could be used include, human Hela, HEK293, H9 and Jurkat cells, mouse NIH3T3 and C127 cells, COS 1, COS 7 and CV1, quail QC1-3 cells, mouse L cells and Chinese hamster ovary (CHO) cells. Also within the scope of the present invention are primary mammalian cells such as mouse embryonic fibroblasts (MEF). Alternatively, the recombinant protein of the invention can be expressed in stable cell' lines that contain the gene construct integrated into a chromosome.

In a more preferred embodiment, said cell is a primary cell or primary cell line. Primary cells are cells which are directly obtained from an organism. Suitable primary cells are, for example, mouse embryonic fibroblasts, mouse primary hepatocytes, cardiomyocytes and neuronal cells as well as mouse muscle stem cells (satellite cells) and stable, immortalized cell lines derived thereof.

Transgenic non-human animals as hosts transfected with and/or expressing the nucleic acid molecule of the present invention also lie within the scope of the invention. In a preferred embodiment, the transgenic animal is a mammal, e.g. a hamster, cow, cat, pig, dog or horse.

Furthermore, the present invention relates to a method for the production of a polypeptide comprising culture of the host cell of the invention under suitable conditions and isolation of the recombinant polypeptide produced.

Suitable conditions for culturing a prokaryotic or eukaryotic host are well known to the person skilled in the art. For example, suitable conditions for culturing bacteria are growing them under aeration in Luria Bertani (LB) medium. To increase the yield and the solubility of the expression product, the medium can be buffered or supplemented with suitable additives known to enhance or facilitate both. E. coli can be cultured from 4 to about 37 °C, the exact temperature or sequence of temperatures depends on the molecule to be overexpressed. In general, the skilled person is also aware that these conditions may have to be adapted to the needs of the host and the requirements of the polypeptide expressed. In case an inducible promoter controls the nucleic acid of the invention in the vector present in the host cell, expression of the polypeptide can be induced by addition of an appriopriate inducing agent. Suitable expression protocols and strategies are known to the skilled person.

Depending on the cell type and its specific requirements, mammalian cell culture can e.g. be carried out in RPMI or DMEM medium containing 10% (v/v) FCS, 2mM L-glutamine and 100 U/ml penicillin/streptomycine. The cells can be kept at 37 °C in a 5% CO₂, water saturated atmosphere.

Suitable media for insect cell culture is e.g. TNM + 10% FCS or SF900 medium. Insect cells are usually grown at 27 °C as adhesion or suspension culture.
Suitable expression protocols for eukaryotic cells are well known to the skilled person and can be retrieved e.g. from in Sambrook, 2001.

Methods of isolation of the polypeptide produced are well-known in the art and comprise without limitation method steps such as ion exchange chromatography, gel filtration chromatography (size exclusion chromatography), affinity chromatography, high pressure liquid chromatography (HPLC), reversed phase HPLC, disc gel electrophoresis or immunoprecipitation, see, for example, in Sambrook, 2001.

In another aspect, the present invention relates to a polypeptide encoded by the nucleic acid molecule of the invention or produced by the method of the invention.

In addition, the present invention relates to a diagnostic composition comprising at least one of the nucleic acid molecule of the invention, the vector of the invention or the polypeptide of the invention.

The enhanced affinity and/or avidity of the polypeptide of the invention enables for its use in diagnostic assays. For example, these molecules can be used as sensitive detection agents for malignant cells, expressing the respective antigens on cell surface. Therefore, mono-specific variants of high avidity, which are equipped with three binding moieties directed against one cell surface antigen can be generated. As another example, tri-specific molecules can be useful for detection of cells expressing at least one of the three antigens the molecule is directed to, with one single agent. Especially the high stability of disulfide stabilized scFv fragments which are incorporated in this format allows a long time storage, which is desirable for diagnostic agents.
Furthermore, the present invention relates to a pharmaceutical composition comprising the nucleic acid molecule of the invention, the vector of the invention or the polypeptide of the invention.

In accordance with the present invention, the term "pharmaceutical composition" relates to a composition for administration to a patient, preferably a human patient. The pharmaceutical composition of the invention comprises the compounds recited above (wherein the term "compound" refers to the mentioned nucleic acid molecule, vector and the polypeptide as well as fragments or derivatives or modifications thereof), alone or in combination. It may, optionally, comprise further molecules capable of altering the characteristics of the compounds of the invention thereby, for example, stabilizing, modulating and/or activating their function. The composition may be in solid, liquid or gaseous form and may be, *inter alia*, in the form of (a) powder(s), (a) tablet(s), (a) solution(s) or (an) aerosol(s). The pharmaceutical composition of the present invention may, optionally and additionally, comprise a pharmaceutical acceptable carrier. By "pharmaceutically acceptable carrier" is meant a non-toxic solid, semisolid or liquid filler, diluent, encapsulating material or formulation auxiliary of any type. Examples of suitable pharmaceutically acceptable carriers are well known in the art and include phosphate buffered saline solutions, water, emulsions, such as oil/water emulsions, various types of wetting agents, sterile solutions, organic solvents including DMSO etc. Compositions comprising such carriers can be formulated by well known conventional methods. The term "parenteral" as used herein refers to modes of administration, which include intravenous, intramuscular, intraperitoneal, intrasternal, subcutaneous and intraarticular injection and infusion. These pharmaceutical compositions can be administered to the subject at a suitable dose. The dosage regimen will be determined by the attending physician and clinical factors. As is well known in the medical arts, dosages for any one patient depend upon many factors, including the patient's size, body surface area, age, the particular compound to be administered, sex, time and route of administration, general health, and other drugs being administered concurrently. The therapeutically effective amount for a given situation will readily be determined by routine experimentation and is within the skills and judgement of the ordinary clinician or physician. Generally, the regimen as a regular administration of the pharmaceutical composition should be in the range of 1 µg to 5 g units per day. However, a more preferred dosage might be in the range of 0.01 mg to 100 mg, even more preferably 0.01 mg to 50 mg and most preferably 0.01 mg to 10 mg per day.

As mentioned above, the nucleic acid molecule of the invention may be formulated into a pharmaceutical composition. The nucleic acid molecule is eventually to be introduced into the desired cells. Appropriate formulations include those wherein 10⁶ to 10¹² copies of the DNA molecule, advantageously comprised in an appropriate vector are administered per dose. The vector may be, for example, a phage, plasmid, viral or retroviral vector. Retroviral vectors may be replication competent or replication defective. In the latter case, viral propagation generally will occur only in complementing host/cells.

The nucleic acid molecule of the invention, the vector of the invention or the polypeptide of the invention can be used for the treatment of tumour. Therefore, the present invention also relates to a method of treating tumour comprising the administration of an amount of the nucleic acid of the invention the vector of the invention or the polypeptide of the invention effective to exert the desired effect to a patient in need thereof.
The desired effect in connection with methods of treatment is inducing an immune response against cells expressing the target tumour antigen(s), especially leukaemia cells.

In a preferred embodiment, the tumours to be treated are leukaemias or lymphomas.
Leukaemias or lymphomas aimed at by the present invention are e.g. AML, CML, ALL, CLL or non-Hodgkin lymphoma.

The figures show:

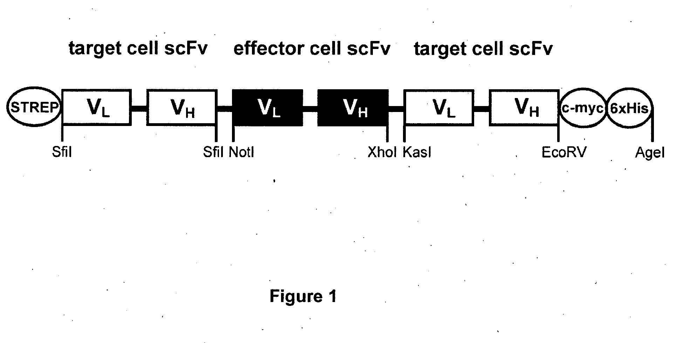

### Figure 1: Construction of the tandem single chain triabody (scFv triabody)

The examples illustrate the invention.

### Example 1: Construction of a tandem scFv triabody

To generate the format of a recombinant tandem single chain Fv triabody (scTb) three scFvs, each comprising a variable light and a variable heavy chain connected via a 20 aa linker (Krebber et al., 1997), have to be fused together with a flexible linker (e.g. 20 aa (G₄S)₄). For the construction of tandem single chain Fv triabodies a multiple cloning site (MCS) has to be ligated into a suitable expression vector, e.g. into the eukaryotic expression vector pSecTag2HygroC (Invitrogen, Karlsruhe, Germany) using apropriate restriction sites. This multiple cloning site and the resulting vector have to provide the restriction sites for the insertion of three scFvs, the linker sequences, tags for purification and detection (e.g. 6xHis, Strep; c-myc), a secretion leader (e.g. Ig kappa chain leader sequence) as well as elements necessary for expression in the chosen system, for eukaryotic expression e.g. a cytomegalus virus (CMV) promotor and an poly-adenylation signal. A resistence cassette (e.g. hygromycin C) will be helpful for the generation of a stably transformed production cell line. The above vector can be genetically engineered in that a first scFv can be inserted as a Sfil cassette, a second scFv as a NotI/XhoI cassette and a third scFv as a KasI/EcoRV cassette. The use of other restriction enzymes is also possible. Each scFv can then be replaced against another scFv, which provides a system for a rapid recombination of scFvs of different specificities (Figure 1). If necessary, disulfide stabilized scFv variants should be used to achieve high serum stability at 37°C.
In this format two scFvs directed against a tumour antigen, e.g. CD19 can be combined with a scFv directed against a effector antigen e.g. CD16, generating scTb [19x16x19]. If necessary, respective scFvs can be amplified using Polymerase Chain Reaction (PCR), thereby introducing the restriction sites needed for cloning them into the vector using standard cloning proceedures (Sambrook & Russel, 2001).

### Example 2: Expression and purification of a tandem scFv triabody

The respective tandem scFv triabody can be expressed in eukaryotic cells e.g. 293T after transient transfection using the calcium phosphate technique including chloroquine (Sambrook & Russel, 2001). Preferably, a production cell line is generated by stable transfection with the linearized (e.g. enzyme Fspl for pSecTag2HygroC) vector coding for this tandem scFv triabody. Positive clones can be selected by e.g. 200µg/ml hygromycin C (Carl Roth, Karlsruhe, Germany) and can subsequently be singularised. For expression, monoclonal stably transfected eukaryotic cells can be cultured under permanent selection in a mini-PERM bioreactor (Greiner Bio-One, Frickenhausen, Germany) with a dialysis membrane of 12.5 kDa in accordance with the manufacturer's instructions. The medium containing recombinant protein has usually to be collected 4 times in a period of two weeks and dialysed at 4°C against a 4000 fold excess of buffer containing 50mM NaH₂PO₄, 300mM NaCl, 10mM Imidazole, pH 8.0. The recombinant tagged proteins can be purified e.g. by affinity chromatography with nickel-nitrilotrioacetic acid (Ni-NTA) agarose beads (Qiagen) and finally dialysed against PBS.

Eluted protein (e.g. scTb[19x16x19]) can be analyzed by reducing Sodium dodecyl sulphate polyacrylamide gel electrophoresis (SDS-PAGE) using standard procedures (Laemmli,1970). Gels are stained with Coomassie brilliant blue R250 (Sigma-Aldrich, Taufkirchen, Germany) will show a protein of approximate Mᵣ = 80-95 kDa. In Western blot analyses, tandem scFv triabodies can be detected using antibodies directed against respective tags, e.g. with an unconjugated penta-His antibody (Qiagen, Hilden, Germany) and secondary horseradish peroxidase (HRP) coupled sheep anti-mouse IgG (Dianova, Hamburg, Germany) antibody, or with HRP conjugated Strep Tag antibody (IBA, Goettingen, Germany). Western Blots are developed using enhanced chemiluminescence reagents (Amersham Pharmacia Biotech, Freiburg, Germany).
This purified antibody derivative can be used for the following experiments:

### Example 3: Determination of antibody equilibrium constants (K_{D}-values)

To asses whether incorporation of a second target cell scFv results in a gain of affinity/avidity, antibody equlilibrium constants (K_{D} values; antibody concentration, at which half maximal binding will be achieved) can be determined. These can be measured by flow cytometry as described previously (Benedict et al, 1997; Bruenke et al., 2004). Immunofluorescence staining should be performed on a FACSCalibur instrument using CellQuest software (Becton Dickinson, Heidelberg, Germany). Briefly, tumour antigen positve cells, e.g CD19 positive SEM cells (Greil et al., 1994) are incubated with increasing concentrations of recombinant antibody fragments on ice. After washing, antibody fragments can be detected using appropriate antibodies e.g. penta-His antibody and phycoerythrin (PE)-conjugated goat anti-mouse IgG (DAKO Diagnostica GmbH, Hamburg, Germany). 1 x 10⁴ events are collected for each sample and whole cells are analysed using appropriate scatter gates to exclude cellular debris and aggregates. Experiments are repeated 6 times and values and graphical analyses are generated with GraphPad Software (Graph Pad software Inc., San Diego, CA, USA) using nonlinear regression curve-fit after normalising maximal fluorescence intensity to 100 %. Group data should be reported as means ± SEM. Differences between K_{D} values are analyzed using unpaired Student's t-test. P-values < 0.05 are considered significant. Likewise, the affinity to the effector cell antigen, e.g. CD16, can be measured. In these experiments the tandem scFv triabody will be compared to a monovalent conventional bsscFv (Bruenkeet al., 2004), consisting of the same scFvs used for construction of the tandem scFv triabody, e.g. one CD19 and one CD16 directed scFv, derived from the same hybridoma. If both tumor cell directed binding moieties are functional and contribute to antigen binding, the tandem scFv triabody should have an higher affinity to the tumour cells as compared to its monovalent counterpart and should exhibit a K_{D} value in low nanomolar ranges. Preferably, the affinity to the effector cell antigen, e.g. CD16, should not be altered by the additional N-terminal fusion of a further scFv. For the effector antigen the tandem scFv triabody should show a K_{D} value that is 4 to 6 times higher as compared to the tumour cell antigen. Hence, the affinity/avidity of this molecule will expectedly be shifted towards the leukaemia cells, as desired.

### Example 4: Effectiveness of the tandem scFv triabody as compared to corresponding bispecific antibody bsscFv.

The tandem scFv triabody can be compared side-by-side to the bsscFv with respect to induction of antibody dependent cytotoxicity (ADCC) by effecor cells, e.g. mononuclear cells (MNCs). ADCC assays with MNCs of healthy donors and tumour derived antigen positive cell lines will be performed in triplicate by a 3-hour ⁵¹Chr release assay as described (Elsasser et al. 1996). At a constant effector to target (E:T) ratio of 40:1 the antibody derivatives are added to the samples in several eqimolar 5-fold serial dilutions and the EC₅₀ values (concentration of antibody derivative that yields 50% of maximal obtained tumour cell lysis) are calculated after substraction of background lysis with Graph-Pad Prism Software by nonlinear regression curve fit. Group data are reported as means ± SEM of at least 4 different experiments. Differences between groups are analyzed using paired Student's t-test. P-values < 0.05 will be considered significant.
The tandem scFv triabody should be capable to induce ADCC and lyse at least three different tumour derived cell lines e.g. SEM in a dose dependent manner with EC₅₀ values in picomolar range. Under identical conditions also the bsscFv antibody should mediate cell lysis, but efficacy is supposed to be considerably lower. The calculated EC₅₀ values are expected to be significantly higher and could be 30 to 40 times higher.
If corresponding data are experimentally confirmed, this will clearly illustrate that under identical experimental conditions the tandem scFv triabody would exhibit a superior lytic activity compared to the bsscFv.

### References:

Ausubel, Current Protocols in Molecular Biology, Green Publishing Associates and Wiley Interscience, N.Y. (2001).
Barbin K, Stieglmaier J, Saul D, Stieglmaier K, Stockmeyer B, Pfeiffer M, Lang P, Fey GH. Influence of variable N-glycosylation on the cytolytic potential of chimeric CD19 antibodies. J Immunother (1997). 2006;29:122-133.
Batra JK, Kasprzyk PG, Bird RE, Pastan I, King CR. Recombinant anti-erbB2 immunotoxins containing Pseudomonas exotoxin. Proc Natl Acad Sci U S A. 1992;89:5867-5871.
Bebbington CR, Renner G, Thomson S, King D, Abrams D, Yarranton GT. High-level expression of a recombinant antibody from myeloma cells using a glutamine synthetase gene as an amplifiable selectable marker. Bio/Technology 1992; 10:169-175.
Benedict CA, MacKrell AJ, Anderson WF. Determination of the binding affinity of an anti-CD34 single-chain antibody using a novel, flow cytometry based assay. J Immunol Methods. 1997; 201 (2):223-31.
Bonnet D and Dick JE. Human acute myeloid leukemia is organized as a hierarchy that originates from a primitive hematopoietic cell. Nat Med. 1997; 3(7): 730-7.
Braasch DA and Corey DR. Locked nucleic acid (LNA): fine-tuning the recognition of DNA and RNA. Chem Biol. 2001; 8(1):1-7.
Brinkmann U, Lee BK and Pastan I. Recombinant Immunotoxins Containing the VH or VL Domain of Monoclonal Antibody B3 fused to Pseudomonas Exotoxin. J. Immunology. 1993; 150:2774-2782.
Bruenke J, Barbin K, Kunert S, Lang P, Pfeiffer M, Stieglmaier K, Niethammer D, Stockmeyer B, Peipp M, Repp R, Valerius T and Fey GH. Effective lysis of lymphoma cells with a stabilised bispecific single-chain Fv antibody against CD19 and FcγRIII (CD16). British Journal of Haematology. 2005; 130:218-228.
Bruenke J, Fischer B, Barbin K, Schreiter K, Wachter Y, Mahr K, Titgemeyer F, Niederweis M, Peipp M, Zunino SJ, Repp R, Valerius T, Fey GH. A recombinant bispecific single-chain Fv antibody against HLA class II and FcgammaRIII (CD16) triggers effective lysis of lymphoma cells. Brit J Haematol. 2004; 125(2): 167-79.
Bryder D, Rossi DJ, Weissman IL. Hematopoietic Stem Cells. Am J Pathol.2006; 169 (2): 338-46.
Carnahan J, Wang P, Kendall R, Chen C, Hu S, Boone T, Juan T, Talvenheimo J, Montestruque S, Sun J, Elliott G, Thomas J, Ferbas J, Kern B, Briddell R, Leonard JP, Cesano A. Epratuzumab, a humanized monoclonal antibody targeting CD22: characterization of in vitro properties. Clin Cancer Res. 2003;9:3982S-3990S.
Cartron G, Dacheux L, Salles G, Solal-Celigny P, Bardos P, Colombat P, Watier H. Therapeutic activity of humanized anti-CD20 monoclonal antibody and polymorphism in IgG Fc receptor FcgammaRIIIa gene. Blood. 2002;99:754-758.
Casasnovas RO, Slimane FK, Garant R, Faure GC, Campos L, Deneys V, Bernier M, Falkenrodt A, Lecalvez G, Maynadie M and Dene MC. Immunological classification of acute myeloplastic leukaemias: relevance to patient outcome. Leukemia. 2003; 17: 515-527.
Castor A, Nilsson L, Astrand-Grundstrom I, Buitenhuis M, Ramirez C, Anderson K, Strombeck B, Garwicz S, Bekassy AN, Schmiegelow K, Lausen B, Hokland P, Lehmann S, Juliusson G, Johansson B, Jacobsen SE. Distinct patterns of hematopoietic stem cell involvement in acute lymphoblastic leukemia. Nat Med. 2005;11:630-637.
Cheung MC, Haynes AE, Stevens A, Meyer RM, Imrie K. Yttrium 90 ibritumomab tiuxetan in lymphoma. Leuk Lymphoma. 2006;47:967-977.
Clynes RA, Towers TL, Presta LG, Ravetch JV. Inhibitory Fc receptors modulate in vivo cytoxicity against tumor targets. Nat Med. 2000;6:443-446.
Davies AJ. Radioimmunotherapy for B-cell lymphoma: Y90 ibritumomab tiuxetan and I(131) tositumomab. Oncogene. 2007;26:3614-3628.
de Palazzo IG, Kitson J, Gercel-Taylor C, Adams S, Weiner LM. Bispecific monoclonal antibody regulation of Fc gamma RIII-directed tumor cytotoxicity by large granular lymphocytes. Cell Immunol. 1992;142:338-347.
Elsasser D, Valerius T, Repp R, Weiner GJ, Deo Y, Kalden JR, van de Winkel JG, Stevenson GT, Glennie MJ, Gramatzki M. HLA class II as potential target antigen on malignant B cells for therapy with bispecific antibodies in combination with granulocyte colony-stimulating factor. Blood. 1996; 87: 3803-12.
Ely P, Wallace PK, Givan AL, Graziano RF, Guyre PM, Fanger MW. Bispecific-armed, interferon gamma-primed macrophage-mediated phagocytosis of malignant non-Hodgkin's lymphoma. Blood. 1996;87:3813-3821.
Eyrich M, Lang P, Lal S, Bader P, Handgretinger R, Klingebiel T, Niethammer D, Schlegel PG. A prospective analysis of the pattern of immune reconstitution in a paediatric cohort following transplantation of positively selected human leucocyte antigen-disparate haematopoietic stem cells from parental donors. Br J Haematol. 2001;114:422-432.
Ghahroudi MA, Desmyter A, Wyns L, Hamers R and Muyldermans S. Selection and identification of single domain antibody fragments from camel heavy-chain antibodies. FEBS Letters 1997; 414:521-526.
Glockshuber R, Malia M, Pfitzinger I, Pluckthun A. A comparison of strategies to stabilize immunoglobulin Fv-fragments. Biochemistry, 1990;29:1362-1367.
Greil J, Gramatzki M, Burger R, Marschalek R, Peltner M, Trautmann U, Hansen-Hagge TE, Bartram CR. Fey GH, Stehr K, et al. The acute lymphoblastic leukaemia cell line SEM with t(4;11) chromosomal rearrangement is biphenotypic and responsive to interleukin-7. Br J Haematol. 1994; 86(2):275-83.
Grossbard ML, Press OW, Appelbaum FR, Bernstein ID, Nadler LM. Monoclonal antibody-based therapies of leukemia and lymphoma. Blood. 1992;80:863-878.
Haagen IA, van deGriend R, Clark M, Geerars A, Bast B, deGast B. Killing of human leukaemia/lymphoma B cells by activated cytotoxic T lymphocytes in the presence of a bispecific monoclonal antibody (α-CD3/α-CD19). Clin. Exp. Immunol. 1992; 90: 368-375.
Hale G, Dyer MJ, Clark MR, Phillips JM, Marcus R, Riechmann L, Winter G, Waldmann H. Remission induction in non-Hodgkin lymphoma with reshaped human monoclonal antibody CAMPATH-1H. Lancet. 1988;2:1394-1399.
Hamann PR, Hinman LM, Beyer CF, Lindh D, Upeslacis J, Flowers DA, Bernstein I. An anti-CD33 antibody-calicheamicin conjugate for treatment of acute myeloid leukemia. Choice of linker. Bioconjug Chem. 2002; 13:40-46.
Harlow and Lane "Antibodies, A Laboratory Manual", Cold Spring Harbor Laboratory Press, 1988.
Harlow and Lane "Using Antibodies: A Laboratory Manual" Cold Spring Harbor Laboratory Press, 1999.
Hartmann F, Renner C, Jung W, Deisting C, Juwana M, Eichentopf B, Kloft M, Pfreundschuh M. Treatment of refractory Hodgkin's disease with an anti-CD16/CD30 bispecific antibody. Blood. 1997;89:2042-2047.
Hekman A, Honselaar A, Vuist WM, Sein JJ, Rodenhuis S, ten Bokkel Huinink WW, Somers R, Rumke P, Melief CJ. Initial experience with treatment of human B cell lymphoma with anti-CD19 monoclonal antibody. Cancer Immunol Immunother. 1991;32:364-372.
Hombach A, Jung W, Pohl C, Renner C, Sahin U, Schmits R, Wolf J, Kapp U, Diehl V, Pfreundschuh M. A CD16/CD30 bispecific monoclonal antibody induces lysis of Hodgkin's cells by unstimulated natural killer cells in vitro and in vivo. Int J Cancer. 1993;55:830-836.
Jin L, Hope KJ, Zhai Q, Smadja-Joffe F, Dick JE. Targeting of CD44 eradicates human myeloid leukemic stem cells. Nat. Med. 2006; 12(10): 1167-74.
Kabat EA, Wu TT. Identical V region amino acid sequences and segments of sequences in antibodies of different specificities. Relative contributions of VH and VL genes, minigenes, and complementarity-determining regions to binding of antibody-combining sites. J Immunol. 1991;147:1709-1719.
Kaminski MS, Zasadny KR, Francis IR, Milik AW, Ross CW, Moon SD, Crawford SM, Burgess JM, Petry NA, Butchko GM, et al. Radioimmunotherapy of B-cell lymphoma with [131I]anti-B1 (anti-CD20) antibody. N Engl J Med. 1993;329:459-465.
Kipriyanov SM, Moldenhauer G, Strauss G and Little M. Bispecific CD3XCD19 diabody for T cell-mediated lysis of malignant human B cells. Int. J. Cancer. 1998; 77: 763-772.
Kipriyanov SM, Cochlovius B, Schafer HJ, Moldenhauer G, Bahre A, Le Gall F, Knackmuss S, Little M. Synergistic antitumor effect of bispecific CD19 x CD3 and CD19 x CD16 diabodies in a preclinical model of non-Hodgkin's lymphoma. J Immunol. 2002;169:137-144.
Krebber A, Bornhauser S, Burmester J, Honegger A, Willuda J; Bosshard HR, Pluckthun A. Reliable cloning of functional antibody variable domains from hybridomas and spleen cell repertoires employing a reengineered phage display system. J Immunol Methods. 1997; 201(1):35-55.
Laemmli UK. Cleavage of structural proteins during the assembly of the head of bacteriophage T4. Nature. 1970; 227(5259): 680-5.
Lang P, Handgretinger R, Niethammer D, Schlegel PG, Schumm M, Greil J, Bader P, Engel C, Scheel-Walter H, Eyrich M, Klingebiel T. Transplantation of highly purified CD34+ progenitor cells from unrelated donors in pediatric leukemia. Blood. 2003;101:1630-1636.
Lapidot T, Sirard C, Vormoor J, Murdoch B, Hoang T, Caceres-Cortes J, Minden M, Paterson B, Caligiuri MA, Dick JE. Nature. 1994; 367 (6464): 645-8.
Legras S, Gunthert U, Stauder R, Curt F, Oliferenko S, Kluin-Nelemans HC, Marie JP, Proctor S, Jasmin C, Smadja-Joffe F. A strong expression of CD44-6v correlates with shorter survival of patients with acute myeloid leukemia. Blood. 1998;91:3401-3413.
Leonard JP, Coleman M, Ketas JC, Chadburn A, Ely S, Furman RR, Wegener WA, Hansen HJ, Ziccardi H, Eschenberg M, Gayko U, Cesano A, Goldenberg DM. Phase I/II trial of epratuzumab' (humanized anti-CD22 antibody) in indolent non-Hodgkin's lymphoma. J Clin Oncol. 2003;21:3051-3059.
Loffler A, Kufer P, Lutterbuse R, Zettl F, Daniel PT, Schwenkenbecher JM, Riethmuller G, Dorken B, Bargou RC. A recombinant bispecific single-chain antibody, CD19 x CD3, induces rapid and high lymphoma-directed cytotoxicity by unstimulated T lymphocytes. Blood. 2000;95:2098-2103.
Messinger Y, Yanishevski Y, Ek O, Zeren T, Waurzyniak B, Gunther R, Chelstrom L, Chandan-Langlie M, Schneider E, Myers DE, Evans W, Uckun FM. In vivo toxicity and pharmacokinetic features of B43 (anti-CD19)-genistein immunoconjugate in nonhuman primates. Clin Cancer Res. 1998;4:165-170.
Murphy G, Cockett MI, Ward RV and Docherty AJ. Matrix metalloproteinase degradation of elastin, type IV collagen and proteoglycan. A quantitative comparison of the activities. Biochem J. 1991; 277:277-279.
Onrust SV, Lamb HM, Balfour JA. Rituximab. Drugs. 1999;58:79-88; discussion 89-90.
Owens GC., Chappell SA., Mauro VP. and Edelman GM. Identification of two short internal ribosome entry sites selected from libraries of random oligonucleotides. Proc. Natl. Acad. Sci. USA 2001; 98(4): 1471-1476.
Pantoliano MW, Bird RE, Johnson S, Asel ED, Dodd SW, Wood JF, Hardman KD. Conformational stability, folding, and ligand-binding affinity of single-chain Fv immunoglobulin fragments expressed in Escherichia coli. Biochemistry. 1991;30:10117-10125.
Passegue E, Jamieson, CHM, Ailles LE, Weissman IL. Normal and leukemic hematopoiesis: Are leukemias a stem cell disorder or a reacquisition of stem cell characteristics? Proc NatI Acad Sci USA. 2003; 100 (suppl.1) 11842-49.
Peipp M, Valerius T. Bispecific antibodies targeting cancer cells. Biochem Soc Trans. 2002;30:507-511.
Pietersz GA, Wenjun L, Sutton VR, Burgess J, McKenzie IF, Zola H, Trapani JA. In vitro and in vivo antitumor activity of a chimeric anti-CD19 antibody. Cancer Immunol Immunother. 1995;41:53-60.
Ravetch JV, Kinet JP. Fc receptors. Annu Rev Immunol. 1991;9:457-492.
Reff ME, Carner K; Chambers KS, Chinn PC, Leonard JE, Raab R, Newman RA, Hanna N, Anderson DR. Depletion of B cells in vivo by a chimeric mouse human monoclonal antibody to CD20. Blood. 1994;83:435-445.
Reiter Y, Brinkmann U, Kreitman RJ, Jung SH, Lee B and Pastan I. Stabilization of the Fv Fragments in Recombinant Immunotoxins by Disulfide Bonds Engineered into Conserved Framework Regions. Biochemistry. 1994; 33:5451-5459.
Riechmann L, Clark M, Waldmann H, Winter G. Reshaping human antibodies for therapy. Nature. 1988;332:323-327.
Hrusak O and Porwit-MacDonald A. Antigen expression patterns reflecting genotype of acute leukaemias. Leukemia. 2002; 16(7):1233-1258.
Sambrook, Molecular Cloning A Laboratory Manual, Cold Spring Harbor Laboratory (2001) N.Y.
Schwemmlein M, Stieglmaier J, Kellner C, Peipp M, Saul D, Oduncu F, Emmerich B, Stockmeyer B, Lang P, Beck JD, Fey GH. A CD19-specific single-chain immunotoxin mediates potent apoptosis of B-lineage leukemic cells. Leukemia. 2007;21:1405-1412.
Segal DM, Weiner GJ, Weiner LM. Bispecific antibodies in cancer therapy. Curr Opin Immunol. 1999;11:558-562.
Sievers EL, Appelbaum FR, Spielberger RT, Forman SJ, Flowers D, Smith FO, Shannon-Dorcy K, Berger MS, Bernstein ID. Selective ablation of acute myeloid leukemia using antibody-targeted chemotherapy: a phase I study of an anti-CD33 calicheamicin immunoconjugate. Blood. 1999;93:3678-3684.
Spangrude GJ, Heimfeld S, Weissman IL. Purification and characterization of mouse hematopoietic stem cells. Science. 1988;241:58-62.
Stemmer WP, Morris SK, Wilson BS. Selection of an active single chain Fv antibody from a protein linker library prepared by enzymatic inverse PCR. Biotechniques. 1993;14:256-265.
Tan BT, Park CY, Ailles E, Weissman IL. The cancer stem cell hypothesis: a work in progress. Lab Invest. 2006; 86: 1203-7.
Taussig DC, Pearce DJ, Simpson C, Rohatiner AZ, Lister TA, Kelly G, Luongo JL, Danet-Desnoyers GH, Bonnet D. Hematopoietic stem cells express multiple myeloid markers: implications for the origin and targeted therapy of acute myeloid leukemia. Blood. 2005;106(13):4086-92.
Vallera DA, Elson M, Brechbiel MW, Dusenbery KE, Burns LJ, Jaszcz WB, Ramsay NK, Panoskaltsis-Mortar A, Kuroki DW, Wagner JE, Vitetta ES, Kersey JH. Radiotherapy of CD19 expressing Daudi tumors in nude mice with Yttrium-90-labeled anti-CD19 antibody. Cancer Biother Radiopharm. 2004;19:11-23.
van de Winkel JG, Anderson CL. Biology of human immunoglobulin G Fc receptors. J Leukoc Biol. 1991;49:511-524.
Vervoordeldonk SF, Merle PA, van Leeuwen EF, von dem Borne AE, Slaper-Cortenbach IC. Preclinical studies with radiolabeled monoclonal antibodies for treatment of patients with B-cell malignancies. Cancer. 1994;73:1006-1011.
Weiner GJ, De Gast GC. Bispecific monoclonal antibody therapy of B-cell malignancy. Leuk Lymphoma. 1995;16:199-207.
Weiner GJ, De Gast GC. Bispecific monoclonal antibody therapy of B-cell malignancy. Leuk Lymphoma. 1995;16:199-207.
Weng WK, Levy R. Two immunoglobulin G fragment C receptor polymorphisms independently predict response to rituximab in patients with follicular lymphoma. J Clin Oncol. 2003;21:3940-3947.

## Claims

1. A nucleic acid molecule encoding a polypeptide, wherein the polypeptide comprises
(a) a first immunoglobulin domain comprising a V_{L} domain linked to a V_{H} domain, wherein the immunoglobulin domain specifically binds to an antigen expressed on tumour cells;
(b) a second immunoglobulin domain comprising a V_{L} domain linked to a V_{H} domain, wherein the immunoglobulin domain specifically binds to an antigen expressed on tumour cells; and
(c) a third immunoglobulin domain comprising a V_{L} domain linked to a V_{H} domain, wherein the immunoglobulin domain specifically binds to an effector cell antigen, wherein the effector cell is selected from the group consisting of NK cells, neutrophilic granulocytes, monocytes and macrophages,
wherein at least one of the antigens expressed on tumour cells and bound by the immunoglobulin domain of (a) or (b) is an antigen expressed on tumour stem cells and/or on tumour precursor or progenitor cells; and
wherein the ratio of immunoglobulin domains binding to antigens expressed on tumour cells and those binding to effector cell antigens is at least 2:1.

2. The nucleic acid molecule of claim 1, wherein the immunoglobulin domain of items (a) and (b) bind to the same antigen expressed on tumour cells.

3. The nucleic acid molecule of claim 1, wherein the immunoglobulin domain of items (a) and (b) bind to different antigens expressed on tumour cells.

4. The nucleic acid molecule of any one of claims 1 to 3, wherein the at least one antigen expressed on tumour stem cells or on tumour precursor or progenitor cells is CD19, CD33, CD13, CD44var or CD123.

5. The nucleic acid molecule of any one of claims 1 to 4, wherein the antigens expressed on tumour cells and bound by the immunoglobulin domains of (a) and (b) are CD19 and CD33, CD19 and CD13, CD19 and CD123, , CD19 and CD44var, CD33 and CD13, CD33 and CD123, CD33 and CD44var, CD13 and CD123, CD13 and CD44var, or CD123 and CD44var.

6. The nucleic acid' molecule of any one of claims 1 to 5, wherein the effector cell antigen bound by the immunoglobulin domain of (c) is CD16 (FcγRIIIa) or CD64 (FcγRI).

7. The nucleic acid molecule of any one of claims 1 to 6, wherein at least one immunoglobulin domain comprises at least one cysteine residue capable of forming intramolecular disulfide bridges.

8. The nucleic acid of any one of claims 1 to 7, wherein the nucleic acid molecule encodes at least one linker which separates at least one V_{H} from a V_{L} domain or at least one V_{L} from a V_{H} domain.

9. The nucleic acid molecule of claim 8, wherein the linker is a peptide linker of at least 5 amino acids.

10. The nucleic acid of claims 8 or 9, wherein the linkers are of uniform length if more than one linker is present.

11. The nucleic acid of claims 8 or 9, wherein the linkers are of different length if more than one linker is present.

12. The nucleic acid of claim 10, wherein the amino acid sequences of the linkers are identical.

13. The nucleic acid of claim 10, wherein the amino acid sequences of the linkers are different.

14. The nucleic acid of any one of claims 1 to 7, wherein no linker is present between at least one V_{H} and V_{L} domain or V_{L} and V_{H} domain.

15. The nucleic acid of any one of claim 1 to 14 encoding two immunoglobulin domains specifically binding to CD19 and one immunoglobulin domain specifically binding to CD16 (FcγRIIIa).

16. The nucleic acid molecule of any one of claims 1 to 15, wherein the polypeptide further comprises at least one (poly)peptide.

17. The nucleic acid molecule of claim 16, wherein the (poly)peptide is a Strep-tag, a His-tag, a Myc-tag, a Flag-tag, a kappa secretion leader, human serum albumin (hsa) or fragments thereof, peptides capable of binding to hsa or other serum proteins; a peptide capable of binding to neonatal Fc receptor (FcRn), human muscle aldolase (hma) or a fragment thereof, CD8 hinge region, an immunoglobulin constant region, Interleukin-2, Interleukin-15 and Interleukin-18, Granulocyte-Macrophage-Colony Stimulating Factor (GM-CSF) or Granulocyte Stimulating Factor (G-CSF).

18. The nucleic acid molecule of any one of claims 1 to 17, wherein the tumour cells are leukaemia cells.

19. The nucleic acid molecule of any one of claims, 1 to 17, wherein the tumour stem cells are leukaemic stem cells.

20. The nucleic acid molecule of any one of claims 1 to 17, wherein the tumour precursor or progenitor cells are leukaemic precursor or progenitor cells.

21. The nucleic acid molecule of any one of claims 1 to 20, wherein the tumour is a leukaemia or lymphoma.

22. A vector comprising the nucleic acid molecule of any one of claims 1 to 21.

23. A non-human host transformed with the vector of claim 22.

24. The non-human host of claim 23, wherein the host is a cell.

25. A method for the production of a polypeptide comprising culture of the host cell of claim 24 under suitable conditions and isolation of the recombinant polypeptide produced.

26. A polypeptide encoded by the nucleic acid molecule of any one of claims 1 to 21 or produced by the method of claim 25.

27. A diagnostic composition comprising at least one of
(a) the nucleic acid molecule of any one of claims 1 to 21
(b) the vector of claim 22 or
(c) the polypeptide of claim 26.

28. A pharmaceutical composition comprising
(a) the nucleic acid molecule of any one of claims 1 to 21
(b) the vector of claim 22 or
(c) the polypeptide of claim 26.

29. The nucleic acid molecule of any one of claims 1 to 21 the vector of claim 22 or the polypeptide of claim 26 for the treatment of tumours.

30. A method for the treatment of tumours comprising the administration of an amount of the nucleic acid of any one of claims 1 to 21 the vector of claim 22 or the polypeptide of claim 26 efficient to exert the desired effect to a patient in need thereof.

31. The method of claim 30, wherein the tumour is a leukaemia or lymphoma.

32. The method of claim 31, wherein the leukaemia is AML, CML, ALL, CLL non-Hodgkin lymphoma.
